# EUROPEAN PATENT APPLICATION

(11) **EP 4 545 009 A1**
(43) Date of publication of application: **30.04.2025**
(21) Application number: 24208047.1
(22) Date of filing: 22.10.2024
(51) Int. Cl.: A61B 6/00

(54) **SUSPENDED TRAVELLING EQUIPMENT AND MEDICAL IMAGING SYSTEM**

(30) Priority: 24.10.2023 CN 202322860352 U
(71) Applicant: Shanghai United Imaging Healthcare Co., Ltd., Shanghai 201807 (CN)
(72) Inventor: LIN, Junwen, Shanghai, 201807 (CN)
(74) Representative: Murgitroyd & Company

(57) **Abstract**

A suspended travelling device and a medical imaging system. The suspended travelling device includes at least one rail (10), at least one guide belt (30), and a movable mechanism (20). The at least one guide belt (30) is arranged on each rail (10) along an extension direction (*X*) of each rail (10). The movable mechanism (20) has at least one first travel roller (21). Each first travel roller (21) is rollable on and abuts against a surface of a corresponding guide belt (30), and a difference between material hardness of an outer peripheral surface of each first travel roller (21) and material hardness of the corresponding guide belt (30) is less than or equal to a preset material hardness threshold.

## Description

### TECHNICAL FIELD

The present disclosure relates to the technical field of medical equipment, and in particular to a suspended travelling device and a medical imaging system.

### BACKGROUND

Suspended digital subtraction angiography (DSA) equipment is usually used in an interventional operating room or a hybrid operating room. Generally, the DSA equipment is suspended from the ceiling of the operating room through an assembly of a movable mechanism and a rail, and the assembly of the movable mechanism and the rail also meets the mobility of the DSA equipment. In order to ensure a movement range of the DSA equipment, the rail is generally configured to be relatively long, and the rail is generally processed and shaped through an aluminum drawing process. However, the length of the rail is relatively large, and thus it is difficult to ensure the uniformity of the spacing between two rails. In addition, in order to ensure the movement capability of the movable mechanism, the surface of the rail for supporting the rollers needs to be flat, while the rollers of the movable mechanism need to be curved-surface supported rollers. The curved-surface supported rollers and the surface of the rail for supporting the rollers engage to ensure the displacement capability of the movable mechanism relative to the rail. However, such an arrangement will cause a large amount of wear between the curved-surface supported rollers of the movable mechanism and the rail processed by the aluminum drawing process to various degrees, thus not only increasing the rolling resistance to the DSA equipment during operation, but also affecting a surgical procedure and cleanliness of the operating room.

### SUMMARY

The present application aims at providing a suspended travelling device and a medical imaging system to solve the problem in the prior art that a relatively large rolling friction between a roller of a movable mechanism and a rail affects movement capability of a DSA equipment and cleanliness of an operating room.

In order to solve the above technical problem, in one aspect, the present invention provides a suspended travelling device, including: at least one rail, at least one guide belt, and a movable mechanism.

Each guide belt is arranged on each rail along an extension direction of each rail.

The movable mechanism has at least one first travel roller. Each first travel roller is rollable on and abuts against a surface of a corresponding guide belt, and a difference between material hardness of an outer peripheral surface of the first travel roller and material hardness of the guide belt is less than or equal to a preset material hardness threshold.

In some embodiments, the material of the outer peripheral surface of the first travel roller and the material of the guide belt are the same.

In some embodiments of the present invention, the at least one rail is two rails (i.e., two rails), and an extension direction of each rail is a straight-line direction, and the two rails are arranged in parallel. The at least one guide belt is two guide belts, and the two guide belts are arranged on the two rails respectively along the extension direction of each rail. The at least one first travel roller is a plurality of first travel rollers, and the plurality of first travel rollers are rollable on and abut against surfaces of the two guide belts respectively.

In some embodiments, each rail has a mounting groove concave in a direction perpendicular to a plane, where each rail is located. The mounting groove extends along the extension direction of each rail. Each guide belt is configured to be inserted into a corresponding mounting groove along the extension direction of each rail, and the mounting groove is configured to engage with and fix the guide belt.

In some embodiments, each rail has an extension part extending toward the movable mechanism. A corresponding guide belt is installed on a first end face of the extension part, and the first end face is an upper end face of the extension part when the suspended travelling device is in use.

In some embodiments, the movable mechanism further includes a plurality of second travel rollers. Each second travel roller is rollable on a second end surface of a corresponding extension part, and the second end surface is a lower end surface of the corresponding extension part when the suspended travelling device is in use.

In some embodiments, the movable mechanism further includes an adjusting shaft. An axis direction of the adjusting shaft is parallel to an axis direction of each of the plurality of first travel rollers. The adjusting shaft is configured to adjust a distance between each second travel roller and the second end surface of the corresponding extension part.

In some embodiments, the movable mechanism has a limiting assembly, and the limiting assembly abuts against two side surfaces of a corresponding extension part to limit an axial movement freedom of the movable mechanism along an axis direction of each first travel roller; the axis direction of each first travel roller is parallel to the corresponding extension part, and perpendicular to the extension direction of each rail.

In some embodiments, the limiting assembly includes a first supporting roller and a second supporting roller, and the first supporting roller and the second supporting roller are configured to abut against the two side surfaces of the corresponding extension part, respectively, and are rollable along the two side surfaces of the corresponding extension part.

In some embodiments, the limiting assembly further includes a supporting-roller seat, and the first supporting roller and the second supporting roller each are installed on the supporting-roller seat.

In some embodiments, an axis direction of the first supporting roller and an axis direction of the second supporting roller each are parallel to a plane, where each rail is located, and are perpendicular to the extension direction of each rail.

In some embodiments, the movable mechanism further includes a restraining shaft; an axis direction of the restraining shaft is parallel to an axis direction of each first travel roller; and two first travel rollers are arranged at two sides of the restraining shaft respectively along the extension direction of each rail.

In some embodiments, the plurality of first travel rollers are evenly distributed on the periphery of the movable mechanism.

In some embodiments, a plurality of supporting seats are arranged on the movable mechanism. Each first travel roller is installed on a corresponding supporting seat through a bearing seat.

Each of the plurality of supporting seats is provided with one of the plurality of first travel rollers. Or each of the plurality of supporting seats is provided with one of the plurality of first travel rollers and one of the plurality of second travel rollers.

In some of embodiments, the plurality of first travel rollers and the plurality of second travel rollers are arranged in a one-to-one correspondence along a direction perpendicular to the first end face and the second end face of the corresponding extension part.

In some embodiments, at least one of an outer peripheral surface of each first travel roller, or the surface of the corresponding guide belt is coated with a lubricating layer.

In some of the embodiments, an outer peripheral surface of each travel roller is coated with a plastic layer.

In another aspect of the present disclosure, the present disclosure further provides a medical imaging system, including any one of the suspended travelling devices above, and an imaging device connected to the movable mechanism of the suspended travelling device.

In some embodiments, the imaging device includes a C-shaped arm, a ray source, and a detector. The arm has two opposite ends, the ray source is installed on one end of the C-shaped arm, and the detector is installed on the other end of the C-shaped arm. The ray source and the detector are aligned with each other.

In some embodiments, the movable mechanism has a mounting hole. The medical imaging system further includes a mounting module connected to the arm of the imaging device. The mounting module is connected to the movable mechanism through the mounting hole on the movable mechanism.

In summary, in the suspended travelling device and the medical imaging system proposed in the present disclosure, the suspended travelling device includes the rail, the guide belt, and the movable mechanism. The rail is fixed on a preset position. The guide belt is arranged on the rail along the extension direction of the rail. The movable mechanism is configured to connect with the imaging device, and has the first travel roller. The first travel roller is rollable on and abuts against the surface of the guide belt. The difference between the material hardness of the outer peripheral surface of the first travel roller and the material hardness of the guide belt is less than or equal to a preset material hardness threshold. In this way, with an engagement of the first travel roller and the guide belt which have the similar material hardness, the wear, which is caused by the friction generated between the first travel roller and the guide belt when the movable mechanism moves along the rail, can be greatly reduced without increasing the resistance to the imaging device during the movement, reducing the generation of debris, and guaranteeing the cleanliness of the operating room.

### BRIEF DESCRIPTION OF THE DRAWINGS

Those skilled in the art should understand that the drawings are provided for a better understanding of the present invention but not intended to limit the scope of the present invention.
FIG. 1 is a view showing a medical imaging system according to an embodiment of the present application;
FIG. 2 is a top view of FIG. 1;
FIG. 3 is a partial side view showing an engagement between a first travel roller and a guide belt according to an embodiment of the present application viewed from a first side;
FIG. 4 is a side view of a suspended travelling device according to an embodiment of the present application;
FIG. 5 is a partial side view showing a limiting assembly of a movable mechanism according to an embodiment of the present application viewed from a second side opposite to the first side.

### Reference numerals:

10. rail; 11. mounting groove; 12. extension part;
20. movable mechanism; 201. mounting hole; 21. first travel roller; 22. second travel roller; 23. adjusting shaft; 24. restraining shaft; 25. limiting assembly; 251. first supporting roller; 252. second supporting roller; 253. supporting-roller seat; 26. supporting seat;
30. guide belt;
40. bearing seat;
50. imaging device; 51. arm; 52. ray source; 53. detector;
60. installing module.

### DETAILED DESCRIPTION OF THE EMBODIMENTS

In order to make the purposes, advantages and features of the present application clearer, the present application is further described in detail below in conjunction with the accompanying drawings and specific embodiments. It should be noted that the drawings are all drawn in a very simplified form and are not drawn to scale, and are only used to assist in explaining the purpose of the embodiments of the present application conveniently and clearly. In addition, structures shown in the drawings are often part of actual structures. In particular, the emphases of the drawings are different, and sometimes different scales are applied.

As used in the present disclosure, the singular forms "one", "an", and "the" include plural objects, the term "or" is generally used to include the meaning of "and/or", the term "various" is generally used to include the meaning of "at least one", and the term "at least two" is generally used to include the meaning of "two or more". In addition, the terms "first", "second", and "third" are used only for descriptive purposes and cannot be understood as indicating or implying relative importance or implicitly indicating the number of technical features indicated. Therefore, the feature defined as "first", "second", or "third" may explicitly or implicitly includes one of the features or at least two of the features. "One end" and "another end" and "proximal end" and "distal end" generally refer to two corresponding parts, which include not only the endpoints. The terms "installed", "connected", and "attached" should be understood in a broad sense, for example, it may be a fixed connection, a detachable connection, or an integral body; it may be a mechanical connection or an electrical connection; it may be a direct connection or an indirect connection through an intermediate medium; and it may be an internal communication between two elements, or an interaction between two elements.

In addition, as used in the present disclosure, an element arranged on another element generally only indicates that there is a connection, coupling, cooperation or transmission relationship between the two elements, and that the connection, coupling, cooperation or transmission between the two elements may be direct or indirect through an intermediate element, and cannot be understood as indicating or implying a spatial position relationship between the two elements, that is, an element may be in any orientation such as inside, outside, above, below or on one side of another element, unless otherwise clearly indicated in the content. For the ordinary skilled in the related art, the specific meanings of the terms in the present disclosure may be understood according to specific circumstances.

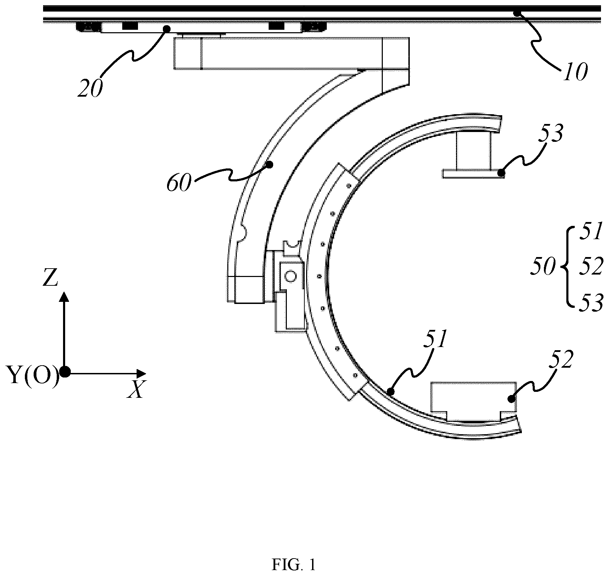

The present invention provides a medical imaging system, and the medical imaging system includes a suspended travelling device, and an imaging device 50 connected to a movable mechanism 20 of the suspended travelling device. FIG. 1 is a view showing a medical imaging system according to an embodiment of the present application, as shown in FIG. 1, the medical imaging system includes an imaging device 50. In an embodiment, the imaging device 50 includes an arm 51, a ray source 52 (i.e., an X-ray source), and a detector 53. The C-shaped arm 51 has two opposite ends, and the ray source 52 is mounted on one end of the C-shaped arm 51, the detector 53 is mounted on the other end of the C-shaped arm 51, and the ray source 52 is aligned with the detector 53. In an embodiment, the arm 51 is a C-shaped arm (as shown in FIG. 1), an O-shaped arm, a G-shaped arm, a straight arm, or a dual-axis hinge mechanism. It may be understood that the ray source 52 is a device capable of emitting X-rays, gamma rays, or electron rays, etc. The detector 53 is a device capable of receiving the rays emitted by the ray source 52. The cooperation of the ray source 52 and the detector 53 may realize an operation such as a medical examination or a treatment. In an embodiment, the ray source 52 includes a tube, which can emit rays (such as X-rays), and the detector 53 may be a flat detector. After passing through a patient, the rays emitted by the tube are received by the detector 53 for imaging processing. The imaging device 50 may be a digital subtraction angiography (DSA) equipment, which may be used in the fields of angiography, cardiology and neurology, which are not limited in the present application. In some embodiments, the imaging device 50 may also be a roller-type equipment, a suspended medical equipment, a suspended surgical robot, etc.

In an embodiment, the present invention provides a suspended travelling device, and the suspended travelling device includes at least one rail 10, at least one guide belt 30 and a movable mechanism 20. The at least one guide belt 30 is arranged on each rail 10 along an extension direction of each rail 10, and the movable mechanism 20 has at least one first travel roller 21. Each first travel roller 21 is rollable on and abuts against a surface of a corresponding guide belt 30, and a difference between material hardness of an outer peripheral surface of each first travel roller 21 and material hardness of the corresponding guide belt 30 is less than or equal to a preset material hardness threshold.

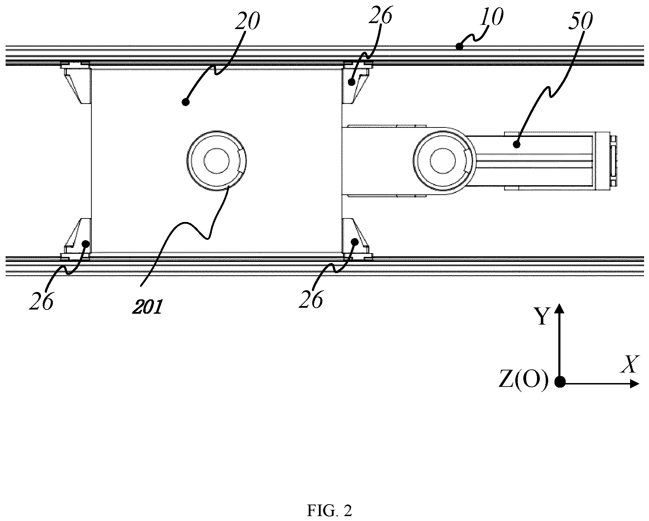

FIG. 2 is a top view of the medical imaging system according to an embodiment of the present application, that is, FIG. 2 is a top view of FIG. 1. Referring to FIG. 1 and FIG. 2, in an embodiment, the medical imaging system further includes a suspended travelling device. The suspended travelling device includes at least one rail 10 (i.e., a hanged rail or a ceiling rail) and a movable mechanism 20 (i.e., an overhead mechanism). Each rail 10 is fixed at a predetermined position, for example, the ceiling of an operating room. In an embodiment, at least two rails 10 are usually arranged in parallel. For example, FIG. 2 shows two rails 10 arranged in parallel along a Y direction, and an extension direction of the rail 10 is usually a straight-line direction, such as an X direction in FIG. 2. The rail may be formed through an aluminum extrusion process, which is convenient to process and shape with a low cost. The movable mechanism 20 is approximately in a shape of a plate, and two sides of the movable mechanism 20 engage with the two rails 10 arranged in parallel, respectively, so that the movable mechanism 20 can move along the rails 10 in the extension direction thereof. The imaging device 50 is mounted on the movable mechanism 20, so that the imaging device 50 can move together with the movable mechanism 20, and so that the imaging device 50 has a larger flexibility. In a specific embodiment, the movable mechanism 20 has a mounting hole 201, and the medical imaging system further includes a mounting module 60 connected to the arm 51 of the imaging device 50. The mounting module 60 is connected to the movable mechanism 20 via the mounting hole 201 on the movable mechanism 20, thereby realizing a connection between the movable mechanism 20 and the imaging device 50. In an embodiment, as shown in FIG. 1, the mounting module 60 includes a first segment and a second segment. A first end of the first segment is connected to the movable mechanism 20 via the mounting hole 201, and a first end of the second segment is arranged on a lower surface of a second end of the first segment, and a second end of the second segment is connected to a middle portion of the arm. In an embodiment, the first segment of the mounting module 60 is configured to be parallel with the extension direction of the rail 10, and the second segment of the mounting module 60 is configured to be parallel with the arm 51. In one of the embodiments, the movable mechanism 20 engages with at least two rails 10, thus ensuring that the imaging device 50 can move together with the movable mechanism 20 more stably.

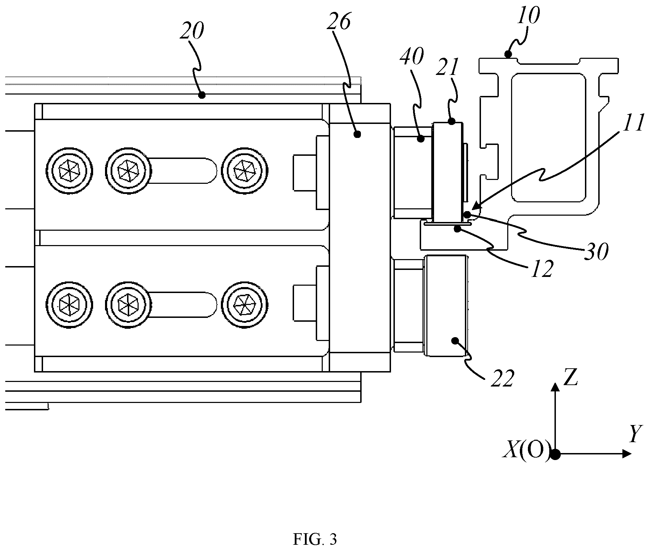

FIG. 3 is a partial side view showing an engagement between a first travel roller and a guide belt according to an embodiment of the present application viewed from a first side. In an embodiment, the at least one guide belt 30 is two guide belts 30, and the two guide belts 30 are arranged on the two rails 10 respectively along the extension direction of each rail 10. The at least one first travel roller 21 is a plurality of first travel rollers 21, and the plurality of first travel rollers 21 are rollable on and abut against surfaces of the two guide belts 30 respectively.

Referring to FIG. 3, in one of the embodiments, in order to realize a movement of the movable mechanism 20 along the extension direction (namely the X direction shown in the figures) of the rail 10, the suspended travelling device further includes at least one guide belt 30, each guide belt 30 is arranged on the corresponding rail 10 along the extension direction (namely, the X direction shown in the figures) of the rail 10, and each rail 10 is installed with one guide belt 30, so that the movable mechanism 20 can move along the guide belt 30 in the extension direction of the rail 10. The movable mechanism 20 has at least one first travel roller 21(i.e., a first wheel), the axis direction (namely the Y direction shown in the figures) of each first travel roller 21 is parallel to a plane (namely the *XOY* plane shown in FIG.2) where each rail 10 is located, and is perpendicular to the extension direction X of the rail 10. In an embodiment, the number of the first travel rollers 21 is plural, and the first travel rollers 21 are distributed on the periphery of the movable mechanism 20. In an embodiment, the first travel roller 21 abuts against and is rollable on the surface of the guide belt 30, and a difference between the material hardness of the outer peripheral surface of the first travel roller 21 and the material hardness of the guide belt 30 is less than or equal to a preset material hardness threshold. The preset material hardness threshold may be 2%, 5%, 10%, 20%, or 30%, which is set according to an actual requirement or according to a wear condition, such as ten-year wear condition. In an embodiment, the material hardness of the outer peripheral surface of the first travel roller 21 is the same as the material hardness of the guide belt 30. In this way, through an engagement of the first travel roller 21 and the guide belt 30 which have similar material hardness, the wear, which is caused by the friction generated between the first travel roller 21 and the guide belt 30 when the movable mechanism 20 moves along the rail 10, can be greatly reduced, thereby reducing the resistance to the imaging device 50 during the movement, reducing the generation of debris, and guaranteeing the cleanliness of the operating room. In an embodiment, the material of the outer peripheral surface of the first travel roller 21 and the material of the guide belt 30 are the same, for example, both are steel.

In an embodiment of the present application, a lubricating layer may be coated on the outer peripheral surface of the first travel roller 21, and/or a lubricating layer may also be disposed on the surface of the guide belt 30, that is, at least one of an outer peripheral surface of each first travel roller 21, or the surface of the corresponding guide belt 30 is coated with the lubricating layer. thereby further reducing the rolling friction between the first travel roller 21 and the guide belt 30. The material of the lubricating layer is biological grease. In specific implementation, the lubricating layer may be formed by applying the biological grease on the outer peripheral surface of the first travel roller 21 or on the surface of the guide belt 30.

As for specific implementation of installing the guide belt 30 onto the rail 10, the rail 10 has a concave mounting groove 11. In an embodiment, the rail 10 has a mounting groove 11 concave in a direction perpendicular to the plane (namely, the *XOY* plane shown in FIG. 2) where each rail 10 is located, and the mounting groove 11 extends along the extension direction *X* of the rail 10 to form a through groove. The guide belt 30 is inserted into the mounting groove 11 along the extension direction X of the rail 10, so that the mounting groove 11 engages with and fixes the guide belt 30. In an embodiment of the present application, the X direction, the Y direction, and the Z direction are perpendicular to each other.

In a specific embodiment, a plurality of supporting seats 26 are arranged on the movable mechanism 20, and each first travel roller 21 is installed on a corresponding supporting seat 26 through a bearing seat 40. Each of the plurality of supporting seats 26 is provided with one of the plurality of first travel rollers 21, or each of the plurality of supporting seats 26 is provided with one of the plurality of first travel rollers 21 and one of the plurality of second travel rollers 22. As shown in FIG. 3, the plurality of supporting seats 26 are distributed on the periphery of the movable mechanism 20. Each first travel roller 21 is installed on the supporting seat 26 through a bearing seat 40, and then installed on the movable mechanism 20 through the supporting seat 26. In an embodiment, the plurality of first travel rollers 21 and the plurality of second travel rollers 22 are arranged in a one-to-one correspondence along a direction perpendicular to the first end face and the second end face of the corresponding extension part 12. In an embodiment, at least two first travel rollers 21 are arranged on each supporting seat 26 along the extension direction X of the rail 10. In some embodiments, only one first travel roller 21 is arranged on each supporting seat 26, such that the plurality of supporting seats 26 arranged on the periphery of the movable mechanism 20 are in a one-to-one correspondence with the plurality of first travel rollers 21, thereby ensuring the balance of support and the stability of movement of the movable mechanism 20 by means of arranging the first travel roller 21 on each supporting seat 26, and thus preventing the operation effect of the imaging device from being affected. Furthermore, the multiple first travel rollers 21 are arranged on the periphery of the movable mechanism 20, such that the force exerted on the bearing of a single first travel roller 21 may be reduced, and that the pressure exerted on the first travel roller 21 is reduced, thereby reducing the wear of the first travel roller 21. In an embodiment, the multiple first travel rollers 21 are evenly arranged on the periphery of the movable mechanism 20. Further, in an embodiment, the multiple supporting seats 26 of the multiple first travel rollers 21 are evenly arranged on the periphery of the movable mechanism 20.

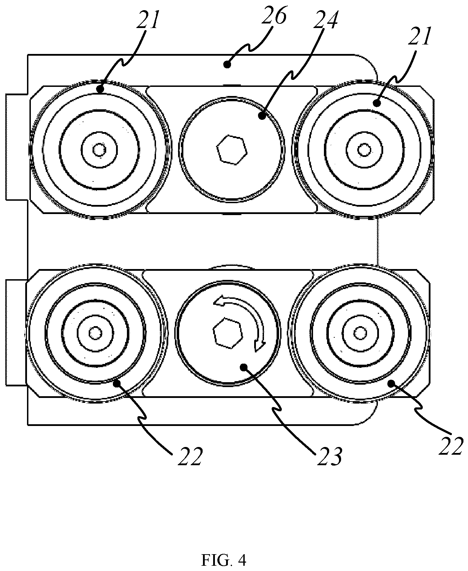

FIG. 4 is a side view of a suspended travelling device according to an embodiment of the present application. Referring to FIG. 4, in an embodiment, the movable mechanism 20 is further provided with a restraining shaft 24, and the axis direction of the restraining shaft 24 is parallel to the axis direction of the first travel roller 21, and two first travel rollers 21 are arranged at opposite two sides of the restraining shaft 24 respectively in the extension direction Xof the rail 10, so that the first travel rollers 21 rotate around the center of the restraining shaft 24 and are prevented from moving along the Y direction. The restraining shaft 24, just as the first travel rollers 21, may be arranged on the supporting seat 26. In this arrangement, the first travel rollers 21 are arranged at opposite two sides of the restraining shaft 24 respectively, such that the force exerted on each first travel roller 21 of the movable mechanism 20 can be reduced, and that the wear of the guide belt 30 caused by the first travel roller 21 can be reduced.

Referring to FIG. 3, in an embodiment, the rail 10 has an extension part 12 extending toward the movable mechanism 20 The guide belt 30 is arranged on a first end face of the extension part 12. In actual application scenarios, the guide belt 30 is arranged on an upper end face of the extension part 12 when the suspended travelling device is in use. The first end face is the upper end face of the extension part 12, namely, the top face of the extension part 12. In an embodiment, the extension part 12 is further configured to extend along the extension direction *X* of the rail 10 to form a plate-shaped part. It should be understandable that end faces (including the first end face and the second face) of the guide belt 30 refer to the surfaces of the guide belt 30, which are parallel to the extension direction Xof the rail 10. The arrangement of the extension part 12 makes it convenient to form the mounting groove 11 on the extension part 12, thus facilitating the engagement between the first travel roller 21 and the guide belt 30. In an embodiment, in an actual application scenario, the end faces of the extension part 12 are parallel to the plane (the *XOY* plane shown in FIG. 2) where each rail 10 is located, so that the guide belt 30, after being installed on the rail 10, is parallel to the plane where each rail 10 is located, thereby enabling a stable operation of the first travel roller 21.

In an embodiment of the present invention, the movable mechanism further comprises a plurality of second travel rollers 22. Each second travel roller 22 is rollable on a second end surface of a corresponding extension part 12, and the second end surface is a lower end surface of the corresponding extension part 12 when the suspended travelling device is in use. Referring to FIG. 3 and FIG. 4, in an embodiment of the present application, a second travel roller 22 (i.e., a second wheel) is further arranged on the movable mechanism 20. The axis direction of the second travel roller 22 is parallel to the axis direction of the first travel roller 21, and the second travel roller 22 is rollable on the second end face of the extension part 12. In actual application scenarios, the second travel roller 22 is rollable on the lower end face of the extension part 12, and in this case, the second end face is the lower end face of the extension part 12, namely the bottom face of the extension part 12. In an embodiment, the second travel roller 22 may be further configured to abut against and rollable on the second end face of the extension part 12. The first travel roller 21 and the second travel roller 22 are arranged along a direction perpendicular to the first end face and the second end face of the extension part 12, that is, the second travel roller 22 is arranged under the first travel roller 21. The second travel roller 22 may be installed on the supporting seat 26. The second end surface of the extension part 12 is constrained by the second travel roller 22, such that the movable mechanism 20 may be prevented from tilting due to the gravity center of the imaging device 50 not being under the gravity center of the movable mechanism 20, thereby ensuring the normal operation of the imaging device 50. In addition, a second travel roller 22 is correspondingly arranged under each of the first travel rollers 21, thus ensuring the forces exerted on the movable mechanism 20 more balanced.

In an embodiment of the present application, the movable mechanism 20 further includes an adjusting shaft 23. The axis direction of the adjusting shaft 23 is parallel to the axis direction of the first travel roller 21, and the adjusting shaft 23 is configured to adjust a distance between the second travel roller 22 and the second end surface of the extension part 12. In an embodiment, the adjusting shaft 23 is an adjustable eccentric shaft, two second travel rollers 22 are arranged on each supporting seat 26, and the adjusting shaft 23 is disposed between the two second travel rollers 22. The arrangement of the adjusting shaft 23 makes it convenient, on one hand, to adjust the second travel roller 22 according to the thickness of the extension part 12 between the two end surfaces thereof, and one the other hand, to install the movable mechanism 20 onto the rail 10.

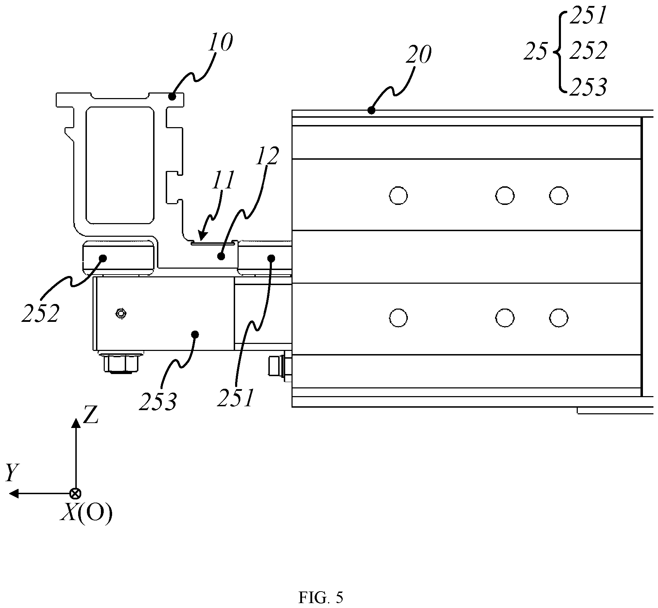

FIG. 5 is a partial side view showing a limiting assembly of a movable mechanism according to an embodiment of the present application viewed from a second side opposite to the first side. Referring to FIG. 5, in an embodiment of the present application, the movable mechanism 20 has a limiting assembly 25. The limiting assembly 25 abuts against two side surfaces of the extension part 12, so that the movement freedom of the movable mechanism 20 is limited in a direction parallel to the axis direction of the first travel roller 21, namely, the *Y* direction shown in the figures. The y direction is parallel to the extension part 12 and perpendicular to the extension direction X of the rail 10. The limiting assembly 25 may limit the position of the movable mechanism 20 in the axis direction of the first travel roller 21, namely, the Y direction. In actual application scenarios, the arrangement of the limiting assembly 25 can avoid a lateral movement of the imaging device 50 after the imaging device 50 is installed on the movable mechanism 20, thereby ensuring the stable operation of the imaging device 50.

In an embodiment, the limiting assembly 25 includes a first supporting roller 251 (i.e., a first supporting wheel) and a second supporting roller 252 (i.e., a second supporting wheel). Further, in an embodiment, both the axis direction of the first supporting roller 251 and the axis direction of the second supporting roller 252 are parallel to the plane (the XOYplane in FIG. 2) where each rail 10 is located, or are perpendicular to the extension direction X of the rail 10. The first supporting roller 251 and the second supporting roller 252 are configured to abut against two side surfaces of the extension part 12 respectively, and are rollable along the two side surfaces of the extension part 12 respectively. In an embodiment, the limiting assembly 25 further includes a supporting-roller seat 253, and the first supporting roller 251 and the second supporting roller 252 are both mounted on the supporting-roller seat 253. In an embodiment, the side surfaces of the extension part 12 are perpendicular to the axis direction of the first travel roller 21, namely, the Y direction in the figure. In an embodiment, the first supporting roller 251 arranged on one side of the suspended travelling device, compared with the second supporting roller 252 arranged on the same side of the suspended travelling device, is closer to the rail 10 arranged on the other side of the suspended travelling device, or is closer to the center of the movable mechanism 20. In an embodiment, the second supporting roller 252 is an adjustable eccentric wheel, and the distance between the first supporting roller 251 and the second supporting roller 252 may be adjusted according to the dimension of the extension part 12 in the axis direction of the first travel roller 21, namely, the Y direction in the figure, thus ensuring that the extension part 12 is located between the first supporting roller 251 and the second supporting roller 252.

In an embodiment of the present application, at least one of an outer peripheral surface of each of the second travel roller 22, an outer peripheral surface of the first supporting roller 251, or an outer peripheral surface of the second supporting roller 252 is coated with a lubricating layer. The lubricating layer may be a plastic layer, or any other material layer that can take functions of lubricating. Specifically, the outer spherical surfaces of these three rollers each are coated with engineering plastics, and the self-lubricating property of the engineering plastics is utilized to reduce the wear of these three rollers.

## Claims

1. A suspended travelling device, **characterized by** comprising:
at least one rail (10);
at least one guide belt (30) arranged on each rail (10) along an extension direction (X) of each rail (10); and
a movable mechanism (20) having at least one first travel roller (21), wherein each first travel roller (21) is rollable on and abuts against a surface of a corresponding guide belt (30), and a difference between material hardness of an outer peripheral surface of each first travel roller (21) and material hardness of the corresponding guide belt (30) is less than or equal to a preset material hardness threshold.

2. The suspended travelling device according to claim 1, wherein the material of the outer peripheral surface of each first travel roller (21) and the material of the corresponding guide belt (30) are the same.

3. The suspended travelling device according to claim 1 or 2, wherein
the at least one rail (10) is two rails (10), and an extension direction of each rail (10) is a straight-line direction, and the two rails (10) are arranged in parallel;
the at least one guide belt (30) is two guide belts (30), and the two guide belts (30) are arranged on the two rails (10) respectively along the extension direction of each rail (10);
the at least one first travel roller (21) is a plurality of first travel rollers (21), and the plurality of first travel rollers (21) are rollable on and abut against surfaces of the two guide belts (30) respectively.

4. The suspended travelling device according to any one of claims 1 to 3, wherein
each rail (10) has a mounting groove (11) concave in a direction perpendicular to a plane (*XOY*) where each rail (10) is located;
the mounting groove (11) extends along the extension direction (X) of each rail (10); and
each guide belt (30) is configured to be inserted into a corresponding mounting groove (11) along the extension direction of each rail (10), and the mounting groove (11) is configured to engage with and fix the guide belt (30).

5. The suspended travelling device according to claim 4, wherein
each rail (10) has an extension part (12) extending toward the movable mechanism (20);
a corresponding guide belt (30) is installed on a first end face of the extension part (12), and the first end face is an upper end face of the extension part (12) when the suspended travelling device is in use.

6. The suspended travelling device according to claim 5, wherein
the movable mechanism (20) further comprises a plurality of second travel rollers (22);
each second travel roller (22) is rollable on a second end surface of a corresponding extension part (12), and the second end surface is a lower end surface of the corresponding extension part (12) when the suspended travelling device is in use.

7. The suspended travelling device according to claim 6, wherein
the movable mechanism (20) further comprises an adjusting shaft (23);
an axis direction of the adjusting shaft (23) is parallel to an axis direction of each of the plurality of first travel rollers (21); and
the adjusting shaft (23) is configured to adjust a distance between each second travel roller (22) and the second end surface of the corresponding extension part (12).

8. The suspended travelling device according to any one of claims 5 to 7, wherein:
the movable mechanism (20) has a limiting assembly (25), and the limiting assembly (25) abuts against two side surfaces of a corresponding extension part (12) to limit an axial movement freedom of the movable mechanism (20) along an axis direction of each first travel roller (21); the axis direction of each first travel roller (21) is parallel to the corresponding extension part (12), and perpendicular to the extension direction (X) of each rail (10).

9. The suspended travelling device according to claim 8, wherein the limiting assembly (25) comprises a first supporting roller (251) and a second supporting roller (252), and the first supporting roller (251) and the second supporting roller (252) are configured to abut against the two side surfaces of the corresponding extension part (12), respectively, and are rollable along the two side surfaces of the corresponding extension part (12);
wherein, optionally, the limiting assembly (25) further comprises a supporting-roller seat (253), and the first supporting roller (251) and the second supporting roller (252) each are installed on the supporting-roller seat (253).

10. The suspended travelling device according to claim 9, wherein an axis direction of the first supporting roller (251) and an axis direction of the second supporting roller (252) each are parallel to a plane (*XOY*) where each rail (10) is located, and are perpendicular to the extension direction (X) of each ceiling rail (10).

11. The suspended travelling device according to any one of claims 1 to 10, wherein the movable mechanism (20) further comprises a restraining shaft (24); an axis direction of the restraining shaft (24) is parallel to an axis direction of each first travel roller (21); and two first travel rollers (21) are arranged at two sides of the restraining shaft (24) respectively along the extension direction (X) of each rail (10).

12. The suspended travelling device according to claim 3, wherein the plurality of first travel rollers (21) are evenly distributed on a periphery of the movable mechanism (20).

13. The suspended travelling device according to any one of claims 6 to 12, wherein
a plurality of supporting seats (26) are arranged on the movable mechanism (20);
each first travel roller (21) is installed on a corresponding supporting seat (26) through a bearing seat (40); and
each of the plurality of supporting seats (26) is provided with one of the plurality of first travel rollers (21), or each of the plurality of supporting seats (26) is provided with one of the plurality of first travel rollers (21) and one of the plurality of second travel rollers (22).

14. The suspended travelling device according to any one of claims 1-13, wherein at least one of an outer peripheral surface of each first travel roller (21) or the surface of the corresponding guide belt (30) is coated with a lubricating layer.

15. A medical imaging system, comprising:
the suspended travelling device according to any one of claims 1 to 14; and
an imaging device (50) connected to the movable mechanism (20) of the suspended travelling device.
